(19) Europäisches Patentamt
European Patent Office
Office européen des brevets

(11) Veröffentlichungsnummer: **0 048 993**
**A1**

# (12) EUROPÄISCHE PATENTANMELDUNG

(21) Anmeldenummer: **81107763.5**

(22) Anmeldetag: **30.09.81**

(51) Int. Cl.³: **C 07 C 99/00**, C 07 C 101/16, C 07 C 153/09

(30) Priorität: **01.10.80 DE 3037160**

(43) Veröffentlichungstag der Anmeldung: **07.04.82**
**Patentblatt 82/14**

(84) Benannte Vertragsstaaten: **AT BE CH DE FR GB IT LI NL SE**

(71) Anmelder: **HOECHST AKTIENGESELLSCHAFT,**
**Postfach 80 03 20, D-6230 Frankfurt/Main 80 (DE)**

(72) Erfinder: **Koch, Manfred, Dr., Kreuzheck 2,**
**D-6239 Eppstein/Taunus (DE)**

(54) **Verfahren zur Herstellung von optisch aktiven 2-Anilino-propionsäureestern.**

(57) Herstellung von optisch aktiven 2-Anilinopropionsäureestern in hoher chemischer und optischer Ausbeute durch Umsetzung von optisch aktiven 2-Chlorpropionsäureestern mit einer zwei- bis zehnfachen Molmenge eines Anilins bei Temperaturen von 90 - 130°C in Abwesenheit von Lösungs- oder Verdünnungsmitteln.

EP 0 048 993 A1

0048993

HOECHST AKTIENGESELLSCHAFT HOE 80/F 225 Dr.GM/St

Verfahren zur Herstellung von optisch aktiven 2-Anilinopropionsäureestern

Gegenstand der vorliegenden Erfindung ist ein Verfahren zur Herstellung von optisch aktiven 2-Anilinopropionsäureestern der allgemeinen Formel I,

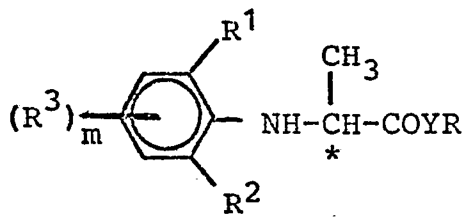

(I)

(* = Chiralitätszentrum)

worin R, $R^1$, $R^2$, $R^3$ gleich oder verschieden sein können und jeweils einen $(C_1-C_4)$-Alkylrest bedeuten, Y für Sauerstoff oder Schwefel und m für die Zahlen O oder 1 stehen, durch Umsetzung eines Chlorpropionsäureesters mit einem Anilin, dadurch gekennzeichnet, daß man optisch aktive 2-Chlorpropionsäureester der allgemeinen Formel II,

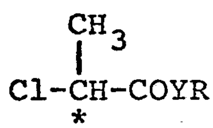

(II)

worin R, Y und * die Bedeutungen wie in Formel I haben, mit einer zwei- bis zehnfachen Molmenge eines Anilins der allgemeinen Formel III,

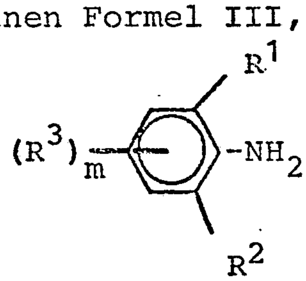

(III)

worin $R^1$, $R^2$, $R^3$ und m die Bedeutungen wie in Formel I haben, bei Temperaturen von 90 - 130°C, vorzugsweise bei 105 - 120°C, in Abwesenheit von Lösungs- oder Verdünnungsmitteln umsetzt.

Es ist aus J. Chem. Soc, Band 1961, S. 3303 - 3308 bekannt, daß man 2,6-Dimethylanilin und 2-Brompropionsäureäthylester in Benzol zu Äthyl-2-/(2,6-dimethylphenyl)-amino/-propionat umsetzen kann, jedoch wird bei einer Umsetzungszeit von 96 Stunden nur eine Ausbeute von 37 % der Theorie erreicht. Durch Anwendung eines dreifachen Überschusses an 2-Brompropionsäureester, bezogen auf 2,6-Dimethylanilin, und Zusatz von Natriumbikarbonat wird die Ausbeute auf 79,6 % verbessert (DE-OS 23 50 944, S. 15, Beispiel 1). Dagegen erhält man mit 2-Brompropionsäureäthylester eine nahezu quantitative Ausbeute, wenn die sterisch hindernden Substituenten in 2,6-Stellung fehlen, d.h., wenn z.B. Anilin als Ausgangsstoff umgesetzt wird. (Chem. Ber. 22 (1889) S. 1792 - 1795).

2-Chlorpropionsäureester zeigen im Vergleich zu entsprechenden 2-Brompropionsäureestern eine wesentlich geringere Reaktivität. Bei der Umsetzung von Anilin mit 2-Chlorpropionsäuremethylester (12 Stunden, 125°C) unter Zusatz von Natriumacetat wird eine Ausbeute an dem gewünschten Anilinopropionester von 40 %, bei der gleichen Umsetzung mit o-Toluidin nur eine Ausbeute von 20 % der Theorie erzielt. (C.R. Acad. Sc. Paris Band 264, Serie C, (1967) S. 1064 - 65). Der starke Einfluß der sterischen Hinderung durch ortho-Substituenten wird bei einer 36-stündigen Umsetzung bei 135°C einer xylolischen Lösung von 2,6-Dimethylanilin mit 2-Chlorpropionsäuremethylester deutlich, bei welcher eine Ausbeute von nur 4,6 % an Methyl-2-/(2,6-dimethylphenyl)-amino/-propionat erreicht wird. Durch Verwendung von im Vergleich zu Xylol für nukleophile Substitutionsreaktionen geeigneteren Lösungsmitteln kann nur eine geringe Verbesserung der Reaktionsausbeute erhalten werden. So wird bei der 60-stündigen Umsetzung von 2-Chlorpropionsäuremethylester mit der 2,5-fachen Molmenge 2,6-Dimethylanilin in Nitrobenzol bei 110°C eine Ausbeute an Anilinopropionsäure von 13 %, unter gleichen Bedingungen in Dimethylformamid

eine Ausbeute von 40 % erzielt.

Bei der Herstellung optisch aktiver Verbindungen der Formel I ist der Erhalt einer hohen optischen Reinheit von besonderer Bedeutung und ein entscheidendes Kriterium. Die üblicherweise angewandte Racemattrennung, ausgehend von racemischem Produkt unter Verwendung einer optisch aktiven Hilfssäure, ist nicht wirtschaftlich durchführbar. Die bei einem solchen Verfahren erforderliche fraktionierende Kristallisation is sehr arbeitsaufwendig, zudem entstehen etwa gleiche Mengen des unerwünschten Enantiomeren.

Die direkte Synthese durch Umsetzung von optisch aktiven Halogenpropionsäureestern mit entsprechenden Anilinen ist nicht bekannt. Diese Art der Herstellung - eine nucleophile Substitution, welche vorwiegend unter Walden'scher Konfigurationsumkehr ablaufen soll - erscheint aus zweifacher Sicht schwierig.

Zum einen sind von den in Frage kommenden 2-Halogenpropionsäureestern nur die enantiomeren Formen der - wie oben gezeigt - reaktionsträgen 2-Chlorpropionsäureester, insbesondere der Methylester, in großtechnischem Maßstab verfügbar, während entsprechende 2-Brompropionsäureester aufgrund der zur Herstellung erforderlichen Spezialreaktionen nur in beschränktem Maße erhältlich bzw. herstellbar sind.

Zum anderen tritt bei der Umsetzung von sowohl optisch aktiven 2-Chlor- wie 2-Brompropionsäureestern mit den entsprechenden Anilinen in erheblichem Umfang Racemisierung auf.

So wird bei der 60-stündigen Umsetzung von L-2-Chlorpropionsäuremethylester mit überschüssigem 2,6-Dimethylanilin in Dimethylformamid bei 110°C racemisches Methyl-2-(N-(2,6-dimethylphenyl)-amino)-propionat erhalten (Ausbeute: 40 % d. Theorie).

Überraschenderweise wurde nun gefunden, daß es durch Umsetzung von optisch aktiven 2-Chlorpropionsäureestern der allgemeinen Formel II mit Anilinen der allgemeinen Formel III in Abwesenheit von Lösungs- oder Verdünnungsmitteln gelingt, die entsprechenden 2-Anilinopropionsäureester der allgemeinen Formel I in hoher chemischer und optischer Ausbeute herzustellen.

Bei dem erfindungsgemäßen Verfahren wird ein optisch aktiver 2-Chlorpropionsäureester der Formel II, bevorzugt der D- oder L-2-Chlorpropionsäuremethylester, mit einem 2 bis 10-molaren, vorzugsweise 2,5 bis 8-molaren Überschuß eines Anilins der Formel III, bevorzugt 2,6-Dimethylanilin, bei Temperaturen von 100 - 130°C, bevorzugt 105 - 120°C, zur Reaktion gebracht, wobei pro Mol Verbindung der Formel II 1 Mol des Anilins+) zur Bindung des freiwerdenden Chlorwasserstoffs unter Salzbildung benötigt wird. Die Reaktionszeiten sind nicht kritisch und betragen im allgemeinen zwischen 36 und 96 Stunden.

+) der Formel III

Bei einer Reaktionszeit von 60 Stunden bei 115°C und einem Molverhältnis 2,6-Dimethylanilin : L-2-Chlorpropionsäuremethylester von 6:1 wird eine Ausbeute von 76 % der Theorie an D-Methyl-2-/N-(2,6-dimethylphenyl)-amino7-propionat erzielt. Die optische Reinheit der erhaltenen Verbindung der Formel I beträgt bei Einsatz von optisch reinem 2-Chlorpropionsäureester zwischen 60 und 85 % d. Theorie.

Nach Beendigung der Umsetzung wird das Hydrochlorid der Verbindung der Formel III auf übliche Weise abgetrennt, entweder durch Rücküberführung in das entsprechende Anilin durch Zusatz einer Base oder durch Waschen mit Wasser. Die Reingewinnung von Verbindungen der Formel I erfolgt dann bevorzugt durch fraktionierte Destillation, bei der überschüssiges Anilin der Formel III als Vorlauf abdestilliert wird.

Die erfindungsgemäß erhältlichen Verbindungen der Formel I stellen wertvolle Zwischenprodukte dar, beispielsweise für die Herstellung von Fungiziden, wie z.B. in den DE-Patentanmeldungen P 29 30 145.9 und P 29 46 910.1 bereits vorgeschlagen.

Die Erfindung wird durch das nachstehende Beispiel näher erläutert.

## Herstellungsbeispiel

### D-Methyl-2-[(N-2,6-dimethylphenyl)amino]-propionat

970 g (8 Mol) 2,6-Dimethylanilin und 122,5 g (1 Mol) L-Methyl-2-chlorpropionat werden 72 Stunden bei 115°C gerührt, anschließend wird das Reaktionsgemisch abgekühlt und mit 300 ml Toluol versetzt. Nach Abfiltrieren von 2,6-Dimethylanilinhydrochlorid wird das Filtrat von Toluol befreit. Durch fraktionierte Destillation erhält man 163 g (= 79 % d. Theorie) D-Methyl-2-[N-(2,6-dimethylphenyl)-amino]-propionat mit dem Siedepunkt 79°C/0,013 mb und einer spezifischen Drehung $[\alpha]_D^{20°}$ = +13,6° (unverdünnt).

Patentansprüche:

1. Verfahren zur Herstellung von optisch aktiven 2-Anilinopropionsäureestern der allgemeinen Formel I,

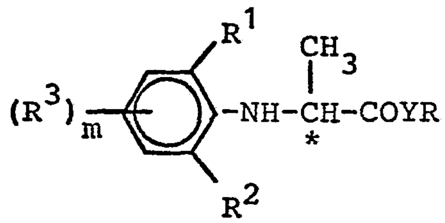

(I)

(* = Chiralitätszentrum)

worin R, $R^1$, $R^2$, $R^3$ gleich oder verschieden sein können und jeweils einen $(C_1-C_4)$-Alkylrest bedeuten, Y für Sauerstoff oder Schwefel und m für die Zahlen O oder 1 stehen, durch Umsetzung eines Chlorpropionsäureesters mit einem Anilin, dadurch gekennzeichnet, daß man optisch aktive 2-Chlorpropionsäureester der allgemeinen Formel II,

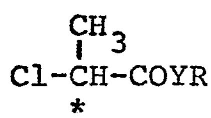

(II)

worin R, Y und * die Bedeutungen wie in Formel I haben, mit einer zwei- bis zehnfachen Molmenge eines Anilins der allgemeinen Formel III,

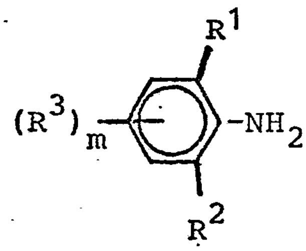

(III)

worin $R^1$, $R^2$, $R^3$ und m die Bedeutungen wie in Formel I haben, bei Temperaturen von 90 - 130°C in Abwesenheit von Lösungs- oder Verdünnungsmitteln umsetzt.

2. Verfahren nach Anspruch 1, dadurch gekennzeichnet, daß man die Umsetzung bei 105 bis 120°C durchführt.

3. Verfahren nach Anspruch 1 oder 2, dadurch gekennzeichnet, daß man einen 2,5 bis 8-molaren Überschuß des Anilins der Formel III einsetzt.

Europäisches Patentamt

# EUROPÄISCHER RECHERCHENBERICHT

0048993

Nummer der Anmeldung
EP 81 10 7763

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - C - 169 358 (B.A.S.F.)<br>* Seite 1, Zeilen 36-57; Beispiel 1 *<br>-- | 1-3 | C 07 C 99/00<br>101/16<br>153/09 |
| | DE - A - 2 447 824 (HERCULES INC.)<br>* Seite 2, Zeile 22 - Seite 4, Zeile 1; Beispiel 1 *<br>-- | 1-3 | |
| | DE - A - 2 513 730 (CIBA-GEIGY A.G.)<br>* Seite 13, Zeile 22 - Seite 14 *<br>-- | 1-3 | RECHERCHIERTE SACHGEBIETE (Int. Cl.³)<br>C 07 C 99/00<br>101/16<br>153/09 |
| | DE - A - 2 650 434 (SHELL)<br>* Ansprüche; Seite 7, Zeile 27 - Seite 8, Zeile 5; Beispiele *<br>---- | 1-3 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 21-12-1981 | PAUWELS |